# EUROPÄISCHE PATENTANMELDUNG

(11) **EP 4 485 707 A1**
(43) Veröffentlichungstag der Anmeldung: **01.01.2025**
(21) Anmeldenummer: 24183283.1
(22) Anmeldetag: 20.06.2024
(51) Int. Cl.: H01R 13/627, H01R 4/28, H01R 4/48, H01R 11/22

(54) **KLEMMVORRICHTUNG UND SENSORKABEL**

(30) Priorität: 30.06.2023 DE 102023117378
(71) Anmelder: Drägerwerk AG & Co. KGaA, 23558 Lübeck (DE)
(72) Erfinder: Graßl, Thomas, 23558 Lübeck (DE)

(57) **Zusammenfassung**

Die vorliegende Erfindung betrifft eine Klemmvorrichtung sowie ein Sensorkabel mit einer solchen Klemmvorrichtung.

## Beschreibung

Die vorliegende Erfindung betrifft eine Klemmvorrichtung sowie ein Sensorkabel mit einer solchen Klemmvorrichtung.

Es ist bekannt, Kabel über eine Klemmvorrichtung mit einem elektrischen Kontakt, wie dem einer Elektrode, elektrisch zu verbinden. Hierfür weisen herkömmliche Elektroden normalerweise eine elektrisch leitende Niete auf, mit welcher die Klemmvorrichtung verbindbar ist. Ein anderes Ende des Kabels ist beispielsweise zum Verbinden mit einem Diagnosegerät, wie beispielsweise einem Patientenmonitor, einem EKG-Gerät, einem EEG-Gerät oder dergleichen ausgebildet und weist hierfür beispielsweise einen Stecker auf. Mittels des Diagnosegeräts ist beispielsweise eine Potenzialdifferenz zwischen zwei Elektroden detektierbar und für eine Bedienperson des Diagnosegeräts visuell und/oder akustisch wahrnehmbar ausgebbar.

Aus der Druckschrift EP 3 797 688 A1 ist eine gattungsgemäße Klemmvorrichtung bekannt. Diese Klemmvorrichtung ist ausgebildet, dass durch ein beidseitiges Drücken gegen eine Rahmenaußenfläche auf einer ersten Rahmenachse ein relatives Bewegen einer Gegenhaltevorrichtung zu einer Anlagevorrichtung in eine erste Freigabeposition zum Freigeben des elektrischen Kontakts aus dem Aufnahmeraum bewirkbar ist, und dass durch ein beidseitiges gegen die Rahmenaußenfläche auf einer von der ersten Rahmenachse verschiedenen zweiten Rahmenachse ein relatives Bewegen der Gegenhaltevorrichtung zur Anlagevorrichtung in eine von der ersten Freigabeposition verschiedene zweite Freigabeposition zum Freigeben des elektrischen Kontakts aus dem Aufnahmeraum bewirkbar ist.

Diese Klemmvorrichtung weist gegenüber weiteren aus den Druckschriften US 9,226,680 B, DE 196 43 988 C1, US 4,040,697 A, US 6,397,439 B, DE 40 09 938 A1, US 2011 151 728 A und US 2004 203 273 A bekannten Klemmvorrichtung vorteilhaft verringerte Herstellkosten sowie eine verbesserte Handhabung auf. Jedoch ist die Handhabung dieser Klemmvorrichtung, obwohl verbessert, nicht durch jede Bedienperson intuitiv erfassbar.

Der vorliegenden Erfindung liegt daher die Aufgabe zu Grunde, eine Klemmvorrichtung sowie ein entsprechendes Sensorkabel bereitzustellen, welche die Nachteile der vorgenannten Klemmvorrichtungen zumindest teilweise nicht aufweisen. Insbesondere liegt der vorliegenden Erfindung liegt daher die Aufgabe zu Grunde, eine Klemmvorrichtung sowie ein entsprechendes Sensorkabel bereitzustellen, deren Handhabung weiter verbessert ist.

Diese und weitere Aufgaben werden gelöst durch eine Klemmvorrichtung mit den Merkmalen des unabhängigen Patentanspruchs sowie durch ein Sensorkabel mit den Merkmalen des nebengeordneten Patentanspruchs.

Weitere Merkmale und Details der Erfindung ergeben sich aus den abhängigen Patentansprüchen, der Beschreibung und den Figuren.

Merkmale und Details, die im Zusammenhang mit der erfindungsgemäßen Klemmvorrichtung beschrieben sind, gelten auch im Zusammenhang mit dem erfindungsgemäßen Sensorkabel und jeweils umgekehrt, sodass bezüglich der Offenbarung zu den einzelnen Erfindungsaspekten stets wechselseitig Bezug genommen werden kann.

Erfindungsgemäß wird eine Klemmvorrichtung zum elektrischen Verbinden eines Kabels mit einem elektrischen Kontakt bereitgestellt. Die Klemmvorrichtung weist ein Halteelement mit einer Haltefläche sowie ein Gegenhalteelement mit einer Gegenhaltefläche auf. Die Haltefläche und die Gegenhaltefläche sind eingerichtet, den elektrischen Kontakt klemmend dazwischen aufzunehmen. Das Halteelement ist durch eine Betätigung, insbesondere durch eine auf das Halteelement drückende Betätigung, mit einer Betätigungskraft derart elastisch verformbar, dass eine Position der Haltefläche relativ zur Gegenhaltefläche zwischen einer ersten Position zur klemmenden Aufnahme des elektrischen Kontakts und einer zweiten Position zu einer Freigabe des elektrischen Kontakts veränderlich ist. Das Halteelement weist einen Schnapppunkt auf, so dass bei einer Betätigung des Halteelements eine der Betätigungskraft entgegenwirkende Rückstellkraft bis zum Erreichen des Schnapppunkt ansteigt, wobei bei Erreichen des Schnapppunkt die Haltefläche schlagartig von der ersten Position in die zweite Position wechselt und die Rückstellkraft abfällt.

In anderen Worten stellt die vorliegende Erfindung eine Klemmvorrichtung bereit, bei der die Haltefläche und die Gegenhaltefläche durch einen Knackfrosch-Mechanismus relativ zueinander positionsveränderlich sind. In noch anderen Worten erfolgt ein Wechsel zwischen der ersten Position und der zweiten Position folglich bei Erreichen einer bestimmten Betätigungskraft, welche den Schnapppunkt kennzeichnet, schlagartig, also schnappend. Diese Wirkungen des Schnappens oder der schlagartigen Positionsveränderung der durch das Halteelement gebildeten Haltefläche beim Betätigen mit ausreichender Betätigungskraft bis zum Schnapppunkt und der plötzliche Abfall der gegen die Betätigungskraft wirkenden Rückstellkraft des Halteelements, ist als "Knackfrosch"-Effekt bekannt, welcher durch den erfindungsgemäßen Knackfrosch-Mechanismus bereitgestellt wird.

Der beim Verformen des Halteelements wenigstens haptisch, vorzugsweise ferner akustisch durch die betätigende Bedienperson wahrnehmbare Knackfrosch-Effekt stellt insofern eine Rückmeldung über das Erreichen des Schnapppunkts und somit über das erfolgende Öffnen der Klemmvorrichtung, also ein Wechsel von der zweiten Position in die erste Position dar. Die Bedienperson erhält somit eine unmittelbare Rückmeldung, dass das Öffnen der Klemmvorrichtung erfolgt ist. Eventuelle Unsicherheiten, ob die aufgebrachte Betätigungskraft bereits zum Öffnen der Klemmvorrichtung ausreicht, können so beseitigt werden. Nach dem Entlasten des Halteelements ist ebenso das Schließen der Klemmvorrichtung, also ein Wechsel von der ersten Position in die zweite Position haptisch und ggf. ferner akustisch wahrnehmbar.

Die vorliegende Erfindung ist also gegenüber bekannten Klemmvorrichtungen insbesondere dahingehend verbessert, dass sich bei herkömmlichen Klemmvorrichtungen ein vergleichsweise gleichmäßiger Kraft/Weg-Verlauf beim Betätigen ergibt, also eine gewissermaßen "analoge" Betätigung erfolgt. Die erfindungsgemäße Bereitstellung einer Klemmvorrichtung mit Knackfrosch-Effekt erlaubt hingegen eine gewissermaßen "digitale" Betätigung. Dies ermöglicht der Bedienperson die klare Unterscheidung zweier Betätigungszustände, nämlich der Zustände "geöffnet", also dass die erste Position vorliegt und "geschlossen", also dass die zweite Position vorliegt. Unerwünschte Zwischenstellungen können vermieden werden.

Weiter vorteilhaft an der erfindungsgemäßen Bereitstellung des Knackfrosch-Effekts ist, dass nach Überschreiten des Schnapppunkts nur noch eine geringe Rückstellkraft gegen die Betätigungskraft wirkt, so dass die Bedienperson das Halteelement mit geringem Kraftaufwand im geöffneten Zustand zur Anbringung an dem elektrischen Kontakt oder zur Entfernung vom elektrischen Kontakt halten kann. Dies reduziert im zeitlichen Mittel die Belastung der Bedienperson gegenüber bekannten Klemmvorrichtungen.

Die vorliegende Erfindung ist nicht auf den Bereich der Medizintechnik beschränkt, sondern in allen technischen Bereichen anwendbar, in denen eine klemmende Aufnahme eines elektrischen Kontakts gewünscht ist. Ein weiteres Anwendungsfeld ergibt sich beispielsweise in der Messtechnik zur Verbindung eines elektrischen Kontakts mit einem elektrischen Messgerät.

Unter einem Halteelement wird ein Teil der Klemmvorrichtung verstanden, welcher eine Haltefläche bereitstellt, also eine Fläche, welche zur, vorzugsweise hintergreifenden, Anlage an den oder um den elektrischen Kontakt geeignet ist.

Unter einem Gegenhalteelement wird ein Teil der Klemmvorrichtung verstanden, welcher eine Gegenhaltefläche bereitstellt, also eine Fläche, welche zur, vorzugsweise hintergreifenden, Anlage an den oder um den elektrischen Kontakt geeignet ist.

Das Halteelement und das Gegenhalteelement können als separate Bauteile einer Baugruppe bereitgestellt sein, welche zur Klemmvorrichtung verbunden sind. Alternativ können das Halteelement und das Gegenhalteelement als jeweilige Bereiche einer integral ausgestalteten Klemmvorrichtungen ausgebildet sein.

Beispielsweise kann das Halteelement aus einer, unter Einwirkung einer durch eine Bedienperson aufbringbaren Betätigungskraft, elastisch verformbaren, d.h. weichen Struktur bestehen oder eine solche Struktur umfassen und mit einem in Bezug auf einem durch eine Bedienperson aufbringbare Bedienkraft im Wesentlichen starren Gegenhalteelement in einem Mehrkomponenten-Spritzgießverfahren verbunden sein. In einer weiteren Variante kann das Gegenhalteelement im Wesentlichen starr bereitgestellt werden und nach Verbinden mit einer Flachfeder, wie einem Federblech, zusammen mit einer Weichkomponente in einem Spritzgießverfahren umspritzt werden. In noch einer weiteren Variante kann zuerst ein Kabel mit dem Halteelement, vorzugsweise dessen Flachfeder, wenn vorhanden, verbunden und dann in einem darauffolgenden Arbeitsschritt das Halteelement und das Kabel zumindest teilweise als Zugentlastung mit der Weichkomponente in einem Spritzgießverfahren umspritzt werden.

Eine klemmende Aufnahme des elektrischen Kontakts zwischen der Haltefläche und der Gegenhaltefläche bezeichnet eine kraft- und/oder formschlüssige Aufnahme des elektrischen Kontakts zwischen der Haltefläche und der Gegenhaltefläche.

Eine Verformbarkeit durch eine Betätigung mit einer Betätigungskraft bezeichnet eine elastische Verformbarkeit des Halteelements, welche durch eine von einer Bedienperson aufbringbare Betätigungskraft erzielbar ist. Die durch die Bedienperson aufgebrachte Kraft wird als die Betätigungskraft verstanden.

Unter einem Schnapppunkt wird der Verformungszustand des Halteelements verstanden, bei dem die Haltefläche schlagartig von der ersten Position in die zweite Position wechselt und die der Betätigungskraft entgegenwirkende Rückstellkraft abfällt.

Vorzugsweise weist das Halteelement eine Flachfeder auf, welche den Schnapppunkt des Halteelements bereitstellt.

Auf diese Weise kann das elastische Deformationsverhalten des Halteelements besonders gezielt angepasst werden.

Eine Flachfeder bezeichnet eine Feder, welche als eine Biegefeder aus einem im Wesentlichen flächenförmigen Ausgangsmaterial ausgestaltet ist. Die Flachfeder kann durch Umformschritte eine räumliche Erstreckung oder Segmente mit räumlicher Erstreckung aufweisen, wobei eine solche Feder auch als Flachformfeder bezeichnet wird. Beispiele für solche Umformschritte sind Prägen, Biegen und/oder Stanzen. Ein Beispiel für eine Flachfeder ist ein Federblech oder eine Blattfeder. Es ist nicht erforderlich, dass die Flachfeder aus einem metallischen Material gefertigt ist. So kann die Flachfeder auch aus einem Kunststoff gefertigt sein.

Vorzugsweise weist das Halteelement, insbesondere die Flachfeder, einen stabilen Zustand auf, welcher der ersten Position der Haltefläche entspricht, und weist ferner einen metastabilen oder instabilen Zustand auf, welcher der zweiten Position der Haltefläche entspricht.

Auf diese Weise kann vorteilhaft sichergestellt werden, dass das Halteelement bzw. die Flachfeder nach Entlasten, d.h. nach Wegnahme der Betätigungskraft aus dem geöffneten Zustand (zweite Position) in den stabilen geschlossenen Zustand (erste Position) zurückschnappt. Verglichen mit bekannten Halteelementen oder Flachfedern, welche bistabil sind, kann so das Schließverfahren des Halteelements bzw. der Flachfeder verbessert werden.

Vorzugsweise weist das Halteelement, insbesondere die Flachfeder, ein bogenförmiges Segment mit einer Bogenform auf, wobei das Segment so ausgebildet ist, dass es durch die Betätigungskraft entgegen der Bogenform durch den Schnapppunkt biegbar ist und bei Wegnahme der Betätigungskraft aufgrund einer Rückstellkraft des Halteelements, insbesondere der Flachfeder, durch den Schnapppunkt hindurch wieder in die Ursprungsform zurückschnappt.

Es ist somit vorgesehen, dass das Abfallen der Rückstellkraft des Halteelements, insbesondere der Flachfeder, durch eine entsprechend schlagartige Formänderung verursacht wird. Die Formänderung kann nach weiter bevorzugter Lehre mit einem hörbaren Knackton einhergehen.

Vorzugsweise ist das Segment im stabilen Zustand in Bezug auf die Klemmvorrichtung nach außen gekrümmt, und das Segment eingerichtet, sich beim Wechsel von der ersten Position in die zweite Position in Bezug auf die Klemmvorrichtung nach innen zu krümmen.

Somit kann die bevorzugte schlagartige Formänderung erreicht werden, indem das Halteelement, insbesondere die Flachfeder, im Bereich des Segments in Längsrichtung verlaufend entgegen der Richtung der Betätigung konvex nach außen gewölbt ist. Das Halteelement, insbesondere die Flachfeder, kann also in Längsrichtung gewissermaßen wannenartig nach außen gewölbt sein. Beim Betätigen des Halteelements muss also zunächst die wannenartige Vorform bis zum Schnapppunkt durchgedrückt werden, was eine erste, vergleichsweise hohe Betätigungskraft erfordert. Nach dem Überschreiten des Schnapppunkts wirkt der Betätigungskraft nur eine einer Federkraft des Halteelements, insbesondere der Flachfeder, entsprechende Rückstellkraft entgegen, so dass die Bedienperson zur weiteren Verformung oder zur Aufrechterhaltung der Verformung nur noch eine zweite, vergleichsweise geringe Betätigungskraft aufwenden muss.

Es ist bevorzugt, dass der maximal durch Betätigungskraft erreichbare Verformungsweg des Halteelements bzw. der Flachfeder durch einen Anschlag begrenzt ist. Eine Überdehnung des Halteelements bzw. der Flachfeder kann hierdurch vermieden werden.

Vorzugsweise umfasst das Halteelement, insbesondere die Flachfeder, ein elektrisch leitendes Material oder besteht aus einem elektrisch leitenden Material, wobei das Halteelement, insbesondere die Flachfeder, eine elektrische Schnittstelle zwischen dem elektrischem Kontakt und dem Kabel bereitstellt.

Besonders bevorzugt kann das Halteelement selber bzw. die Flachfeder zusätzlich als elektrischer Leiter fungieren, so dass durch diese Funktionsintegration die Anzahl notwendiger Bauelemente zur Bereitstellung der Klemmvorrichtung und folglich die Herstellkosten vorteilhaft verringert werden können.

Ein elektrisch leitendes Material kann beispielsweise ein Metall wie ein Stahl sein, besonders bevorzugt ein Federstahl, ganz besonders bevorzugt ein Federstahl nach EN 10089.

Besonders bevorzugt ist die Flachfeder als Federblech ausgestaltet.

Dabei kann das Federblech wie vorbeschrieben Segmente mit räumlicher Erstreckung aufweisen oder insgesamt räumlich ausgestaltet sein. Insbesondere kann das Federblech ein vorbeschriebenes bogenförmiges Segment aufweisen.

Durch Bereitstellung der Flachfeder als Federblech können die Herstellungskosten der Klemmvorrichtung vorteilhaft verringert werden, da Federbleche kostengünstig erhältlich sind.

Vorzugsweise ist das Halteelement in zwei voneinander beabstandeten Bereichen mit dem Gegenhalteelement lagernd verbunden.

Auf diese Weise kann zwischen den beabstandeten Bereichen eine Betätigungsfläche zur Betätigung mit der Betätigungskraft bereitgestellt und eine Kraftaufnahme der Betätigungskraft durch die Lagerstellen sichergestellt werden.

Eine lagernde Verbindung kann als eine Festlagerung und/oder eine Loslagerung ausgestaltet sein. Eine Bereitstellung von zwei Festlagerungen ist bevorzugt, da spaltfrei möglich. Dies wiederum ist vorteilhaft hinsichtlich der Reinigbarkeit der Klemmvorrichtung.

Vorzugsweise sind die Haltefläche und die Gegenhaltefläche eingerichtet, den elektrischen Kontakt in einer Greifebene zu greifen, wobei die Betätigungskraft auf das Halteelement in einer Betätigungsrichtung aufbringbar ist, und wobei die Betätigungsrichtung parallel zur Greifebene ist.

In anderen Worten wird der elektrische Kontakt seitlich gegriffen. Hierdurch kann sichergestellt werden, dass bei einer Befestigung und bei einem Lösen der Klemmvorrichtung von dem elektrischen Kontakt keine oder nur geringe Druckkräfte außerhalb der Greifebene auf den elektrischen Kontakt ausgeübt werden, welche durch einen Patienten als unangenehm empfunden werden könnten. Folglich kann der Patientenkomfort durch eine derartige Klemmvorrichtung verbessert werden.

In einer bevorzugten Ausgestaltung sind die Haltefläche und die Gegenhaltefläche eingerichtet, den elektrischen Kontakt in einer Mehrzahl von Greifebenen zu greifen, wobei die Betätigungskraft auf das Halteelement in der Betätigungsrichtung aufbringbar ist, und wobei die Betätigungsrichtung parallel zu der Mehrzahl von Greifebenen ist.

Somit ist ein Umgreifen bei "falsch" gegriffener Klemme unnötig, denn sie kann auf beiden Seiten greifen.

Erfindungsgemäß wird ferner ein Sensorkabel zum elektrischen Verbinden eines elektrischen Kontakts mit einem Medizingerät bereitgestellt, das Sensorkabel aufweisend eine Anzahl von Kabeln und eine Anzahl von Klemmvorrichtungen nach einem der vorherigen Ansprüche.

Unter einer Anzahl wird insofern eine Einzahl oder eine Mehrzahl verstanden.

Jedes Kabel kann ein- oder mehrpolig ausgestaltet sein.

Vorzugsweise sind die Anzahl von Kabeln und die Anzahl von Klemmvorrichtungen integral miteinander ausgebildet.

Diese Merkmale und weitere Details der Erfindung ergeben sich auch aus der nachfolgenden Figurenbeschreibung. Dabei zeigt
- **Fig.** 1a: ein Ausführungsbeispiel einer erfindungsgemäßen Klemmvorrichtung in einem geschlossenen Zustand in Seitenansicht,
- **Fig. 1b**: die erfindungsgemäße Klemmvorrichtung nach Fig. 1a in einem geöffneten Zustand in Seitenansicht,
- **Fig. 1c**: die erfindungsgemäße Klemmvorrichtung nach Fig. 1a in dem geschlossenen Zustand in perspektivischer Ansicht,
- **Fig. 2**: ein Ausführungsbeispiel einer erfindungsgemäßen Flachfeder in perspektivischer Ansicht, und
- **Fig. 3**: ein schematisches Weg-Rückstellkraft-Diagramm einer erfindungsgemäßen Klemmvorrichtung.

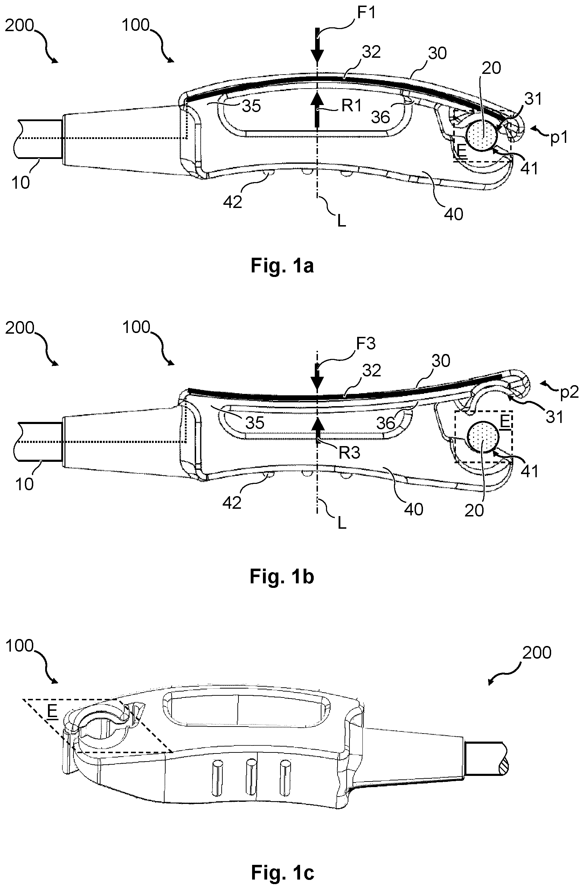

Erfindungsgemäß wird eine Klemmvorrichtung 100 bereitgestellt. Ein Ausführungsbeispiel einer erfindungsgemäßen Klemmvorrichtung 100 ist in den Fig. 1a - 1c dargestellt.

Die dargestellte Klemmvorrichtung 100 dient zum elektrischen Verbinden eines Kabels 10 mit einem elektrischen Kontakt 20. Die Klemmvorrichtung 100 weist ein Halteelement 30 mit einer Haltefläche 31 und ein Gegenhalteelement 40 mit einer Gegenhaltefläche 41 auf. Im dargestellten Beispiel sind das Halteelement 30 und das Gegenhalteelement 40 in einem Mehrkomponenten-Spritzgießverfahren miteinander verbunden bereitgestellt.

Die Haltefläche 31 und die Gegenhaltefläche 41 sind eingerichtet sind, den als schematischen Kreis dargestellten elektrischen Kontakt 20 klemmend dazwischen aufzunehmen. Der elektrische Kontakt 20 kann eine im Wesentlichen beliebige Kontur und räumliche Form aufweisen.

Das Halteelement 30 ist durch eine Betätigung mit einer Betätigungskraft F derart elastisch verformbar, dass eine Position der Haltefläche 31 relativ zur Gegenhaltefläche 41 zwischen einer ersten Position p1 zur klemmenden Aufnahme des elektrischen Kontakts 20 und einer zweiten Position p2 zu einer Freigabe des elektrischen Kontakts 20 veränderlich ist.

In Fig. 1a ist insofern ein geschlossener Zustand der Klemmvorrichtung 100 dargestellt, also ein Zustand, indem die Position der Haltefläche 31 relativ zur Gegenhaltefläche 41 die erste Position p1 ist.

Das Halteelement 30 weist einen Schnapppunkt auf, so dass bei einer Betätigung des Halteelements 30 eine der Betätigungskraft F entgegenwirkende Rückstellkraft R bis zum Erreichen des Schnapppunkts ansteigt und wobei bei Erreichen des Schnapppunkts die Haltefläche 31 schlagartig von der ersten Position p1 in die zweite Position p2 wechselt und die Rückstellkraft R abfällt.

In Fig. 1b ist insofern ein geöffneter Zustand der Klemmvorrichtung 100 dargestellt, also ein Zustand, indem die Position der Haltefläche 31 relativ zur Gegenhaltefläche 41 die zweite Position p2 ist.

Wenn auf das Halteelement 30 im geschlossenen Zustand eine Betätigungskraft F1 aufgebracht wird, welcher geringer ist als die dem Schnapppunkt entsprechende Betätigungskraft F2, findet nur eine geringe Verformung des Halteelements 30 ohne Öffnung der Klemmvorrichtung statt. Erst, wenn die Betätigungskraft F2 die dem Schnapppunkt entsprechende Betätigungskraft F2 erreicht, findet ein schlagartiger Wechsel von der ersten Position p1 in die zweite Position p2 statt, also ein Wechsel von dem in Fig. 1a dargestellten Zustand in den in Fig. 1b dargestellten Zustand. Ein Aufrechterhalten dieses geöffneten Zustands erfordert lediglich eine vergleichsweise geringe Betätigungskraft F3, also eine Betätigungskraft F3, welcher geringer ist als die dem Schnapppunkt entsprechende Betätigungskraft F2.

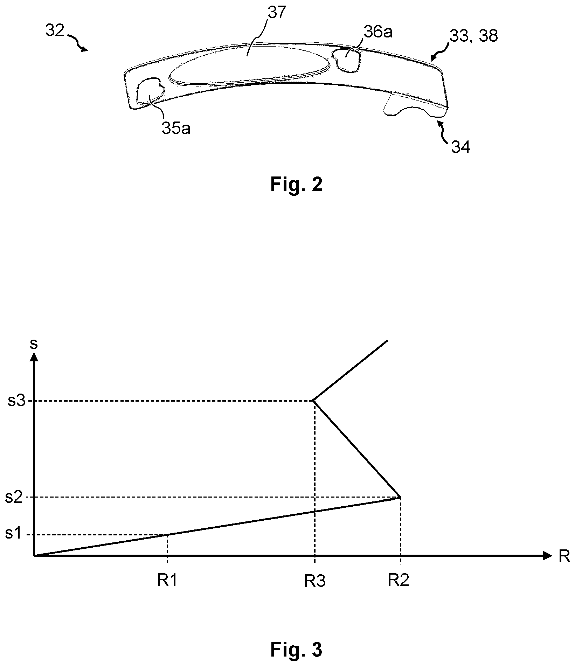

Dieses erfindungsgemäße Verhalten ist in einer abstrahierten Prinzipdarstellung in dem Diagramm nach Fig. 3 verdeutlicht, in welchem der Verformungsweg s des Halteelements 30 gegen die Rückstellkraft R des Halteelements 30 schematisch aufgetragen ist. Bis zum Schnapppunkt, welcher durch eine vorbestimmte Rückstellkraft R2 bei einem vorbestimmten Verformungsweg s2 gekennzeichnet ist, muss eine relativ hohe Betätigungskraft aufgebracht werden, um das Halteelement 30 zu verformen. Ein Verformungsweg s1 mit einer der Rückstellkraft R1 entsprechenden Betätigungskraft F1 löst somit noch kein Schnappen bzw. keine schlagartige Formänderung aus. Mit Erreichen des Schnapppunkts mit dem Verformungsweg s2 und mit einer der Rückstellkraft R2 entsprechenden Betätigungskraft F2 findet eine schlagartige Verformung des Halteelements 30 statt, wodurch der Verformungsweg s schlagartig auf s3 ansteigt und gleichzeitig die Rückstellkraft R3 und somit die zur Aufrechterhaltung des Verformungszustands benötigte Betätigungskraft F3 abfällt.

Das Halteelement 30 kann einen stabilen Zustand aufweisen, welcher der ersten Position p1 der Haltefläche 31 entspricht und ferner einen metastabilen oder instabilen Zustand aufweisen, welcher der zweiten Position p2 der Haltefläche 31 entspricht.

Im dargestellten Ausführungsbeispiel kann das Halteelement 30 eine Flachfeder 32 aufweisen, welche den Schnapppunkt s2, F2 des Halteelements 30 bereitstellt. Die Flachfeder 32 ist im dargestellten Ausführungsbeispiel als Federblech 32 mit sich räumlich erstreckenden Segmenten ausgestaltet, wie dies in Fig. 2 dargestellt ist. So weist die Flachfeder 32 ein bogenförmiges Segment 33 mit einer Bogenform auf. Im dargestellten Ausführungsbeispiel wird die Flachfeder 32 in ihrer Längsrichtung durch das bogenförmige Segment 33 gebildet. Das Segment 33 ist so ausgebildet ist, dass es durch die Betätigungskraft F2 entgegen der Bogenform durch den Schnapppunkt biegbar ist und bei Wegnahme der Betätigungskraft F2 aufgrund der Rückstellkraft der Flachfeder 32 durch den Schnapppunkt hindurch wieder in die Ursprungsform zurückschnappt.

Es ist nicht erforderlich, dass die Flachfeder 32 das bogenförmige Segment 33 aufweist. So kann das Halteelement 30 selber, beispielsweise aus Kunststoff geformt, ein bogenförmiges Segment 33 mit einer Bogenform aufweisen, wobei das Segment 33 so ausgebildet sein kann, dass es durch die Betätigungskraft F entgegen der Bogenform durch den Schnapppunkt s2, F2 biegbar ist und bei Wegnahme der Betätigungskraft F aufgrund einer Rückstellkraft des Halteelements 30 durch den Schnapppunkt s2, F2 hindurch wieder in die Ursprungsform zurückschnappt.

Das Segment 33 nach Fig. 2 ist im stabilen Zustand in Bezug auf die Klemmvorrichtung 100 nach außen gekrümmt und eingerichtet, sich beim Wechsel von der ersten Position p1 in die zweite Position p2 in Bezug auf die Klemmvorrichtung 100 nach innen zu krümmen, wie dies beispielsweise im Vergleich von Fig. 1a zu Fig. 1b deutlich wird.

Im dargestellten Ausführungsbeispiel kann die Flachfeder 32 ein elektrisch leitendes Material umfassen oder aus einem elektrisch leitenden Material bestehen. Ein Beispiel für ein solches Material ist Federstahl.

Wie in Fig. 1a und 1b ersichtlich ist, kann die Flachfeder 32 eine elektrische Schnittstelle 38 zwischen dem elektrischem Kontakt 20 und dem Kabel 10 bereitstellen.

Das elektrisch leitende Material muss jedoch nicht mittels der Flachfeder 32 bereitgestellt sein. So ist es möglich, dass das Halteelement 30 ein elektrisch leitendes Material umfasst oder aus einem elektrisch leitenden Material besteht, und dass das Halteelement 30 die elektrische Schnittstelle 38 zwischen dem elektrischen Kontakt 20 und dem Kabel 10 bereitstellt.

Wie ferner in Fig. 1a und 1b dargestellt ist, kann das Halteelement 30 in zwei voneinander beabstandeten Bereichen, nämlich in einem ersten Lagerbereich 35 und in einem zweiten Lagerbereich 36 mit dem Gegenhalteelement 40 lagernd verbunden sein. Hierzu kann die Flachfeder 32, wie in Fig. 2 dargestellt, zwei beispielsweise gestanzte oder geprägte Bereich aufweisen, welche korrespondierend einen ersten Lagerabschnitt 35a und zweiten Lagerabschnitte 36a bereitstellen.

Die Flachfeder 32 kann, wie in Fig. 2 dargestellt, ferner einen Umformabschnitt 37 aufweisen, um die mechanischen Eigenschaften der Flachfeder 32 noch weiter anzupassen.

Wie in Fig. 1a - 1c ersichtlich ist, sind die Haltefläche 31 und die Gegenhaltefläche 41 eingerichtet, den elektrischen Kontakt 20 in einer Greifebene E zu greifen, also eine Schließ- bzw. Öffnungsbewegung in der Greifebene E auszuführen. In Fig. 1a, 1b liegt die Greifebene E in der Betrachtungsebene, in Fig. 1c erstreckt sich die Greifebene E in die Betrachtungsebene hinein.

Die Betätigungskraft F ist dabei auf das Halteelement 30 in einer Betätigungsrichtung L aufbringbar, wobei die Betätigungsrichtung L parallel zur Greifebene E ist.

Zum Greifen des elektrischen Kontakts 30 kann die Flachfeder 32 beispielsweise ein Greifsegment 34 aufweisen, welches sich von dem bogenförmigen Segment 33 abgewinkelt wegerstrecken kann. Für den bevorzugten Fall, dass die Flachfeder 32 ein elektrisch leitendes Material umfasst oder aus einem elektrisch leitenden Material besteht, bildet das Greifsegment 34 gleichzeitig eine elektrische Kontaktierungsfläche der Haltefläche 31.

Wie in Fig. 1c besonders gut ersichtlich ist, kann auch die Gegenhaltefläche 41 eine Formgebung aufweisen, welche das Greifen des elektrischen Kontakts 30 ermöglicht oder verbessert.

Wie aus Fig. 1a - 1c ersichtlich ist, wird erfindungsgemäß außerdem ein Sensorkabel 200 zum elektrischen Verbinden eines elektrischen Kontakts 20 mit einem Medizingerät bereitgestellt. Das Sensorkabel 200 weist eine Anzahl von Kabeln 10 und eine Anzahl von vorbeschriebenen Klemmvorrichtungen 100 auf.

Die Anzahl von Kabeln 10 und die Anzahl von Klemmvorrichtungen 100 kann dabei integral miteinander ausgebildet sein.

### Bezugszeichenliste

- 10: Kabel
- 20: elektrischer Kontakt
- 30: Halteelement
- 31: Haltefläche
- 32: Flachfeder, Federblech
- 33: bogenförmiges Segment
- 34: Greifsegment
- 35: erster Lagerbereich
- 35a: erster Lagerabschnitt
- 36: zweiter Lagerbereich
- 36a: erster Lagerabschnitt
- 37: Umformabschnitt
- 38: elektrische Schnittstelle
- 40: Gegenhalteelement
- 41: Gegenhaltefläche
- 42: Oberflächenstruktur
- 100: Klemmvorrichtung
- 100: Sensorkabel

- E: Greifebene
- F1, F3: Betätigungskraft
- R, R1, R2, R3: Rückstellkraft
- L: Betätigungsrichtung
- p1: erste Position
- p2: zweite Position
- s, s2: Betätigungsweg

## Patentansprüche

1. Klemmvorrichtung (100) zum elektrischen Verbinden eines Kabels (10) mit einem elektrischen Kontakt (20), aufweisend
- ein Halteelement (30) mit einer Haltefläche (31), und
- ein Gegenhalteelement (40) mit einer Gegenhaltefläche (41),
wobei die Haltefläche (31) und die Gegenhaltefläche (41) eingerichtet sind, den elektrischen Kontakt (20) klemmend dazwischen aufzunehmen,
wobei das Halteelement (30) durch eine Betätigung mit einer Betätigungskraft (F, F2) derart elastisch verformbar ist, dass eine Position der Haltefläche (31) relativ zur Gegenhaltefläche (41) zwischen einer ersten Position (p1) zur klemmenden Aufnahme des elektrischen Kontakts (20) und einer zweiten Position (p2) zu einer Freigabe des elektrischen Kontakts (20) veränderlich ist,
wobei das Halteelement (30) einen Schnapppunkt (s2, F2) aufweist, so dass bei einer Betätigung des Halteelements (30) eine der Betätigungskraft (F, F1) entgegenwirkende Rückstellkraft (R, R1) bis zum Erreichen des Schnapppunkts (s2, F2) ansteigt,
wobei bei Erreichen des Schnapppunkts (s2, F2) die Haltefläche (31) schlagartig von der ersten Position (p1) in die zweite Position (p2) wechselt und die Rückstellkraft (R, R3) abfällt.

2. Klemmvorrichtung (100) nach Anspruch 1,
wobei das Halteelement (30) eine Flachfeder (32) aufweist, welche den Schnapppunkt (s2, F2) des Halteelements (30) bereitstellt.

3. Klemmvorrichtung (100) nach Anspruch 1 oder 2,
wobei das Halteelement (30), insbesondere die Flachfeder (32), einen stabilen Zustand aufweist, welcher der ersten Position (p1) der Haltefläche (31) entspricht, und
wobei das Halteelement (30), insbesondere die Flachfeder (32), einen metastabilen oder instabilen Zustand aufweist, welcher der zweiten Position (p2) der Haltefläche (31) entspricht.

4. Klemmvorrichtung (100) nach einem der vorherigen Ansprüche,
wobei das Halteelement (30), insbesondere die Flachfeder (32), ein bogenförmiges Segment (33) mit einer Bogenform aufweist,
wobei das Segment (33) so ausgebildet ist, dass es durch die Betätigungskraft (F) entgegen der Bogenform durch den Schnapppunkt (s2, F2) biegbar ist und bei Wegnahme der Betätigungskraft (F) aufgrund einer Rückstellkraft des Halteelements (30), insbesondere der Flachfeder (32), durch den Schnapppunkt (s2, F2) hindurch wieder in die Ursprungsform zurückschnappt.

5. Klemmvorrichtung (100) nach Anspruch 4,
wobei das Segment (33) im stabilen Zustand in Bezug auf die Klemmvorrichtung (100) nach außen gekrümmt ist, und
wobei das Segment (33) eingerichtet ist, sich beim Wechsel von der ersten Position (p1) in die zweite Position (p2) in Bezug auf die Klemmvorrichtung (100) nach innen zu krümmen.

6. Klemmvorrichtung (100) nach einem der vorherigen Ansprüche,
wobei das Halteelement (30), insbesondere die Flachfeder (32), ein elektrisch leitendes Material umfasst oder aus einem elektrisch leitenden Material besteht, und
wobei das Halteelement (30), insbesondere die Flachfeder (32), eine elektrische Schnittstelle (38) zwischen dem elektrischen Kontakt (20) und dem Kabel (10) bereitstellt.

7. Klemmvorrichtung (100) nach einem der vorherigen Ansprüche, wobei die Flachfeder (32) als Federblech (32) ausgestaltet ist.

8. Klemmvorrichtung (100) nach einem der vorherigen Ansprüche,
wobei das Halteelement (30) in zwei voneinander beabstandeten Bereichen (35, 36) mit dem Gegenhalteelement (40) lagernd verbunden ist.

9. Klemmvorrichtung (100) nach einem der vorherigen Ansprüche,
wobei die Haltefläche (31) und die Gegenhaltefläche (41) eingerichtet sind, den elektrischen Kontakt (20) in einer Greifebene (E) zu greifen,
wobei die Betätigungskraft (F) auf das Halteelement (30) in einer Betätigungsrichtung (L) aufbringbar ist, und
wobei die Betätigungsrichtung (L) parallel zur Greifebene (E) ist.

10. Sensorkabel (200) zum elektrischen Verbinden eines elektrischen Kontakts (20) mit einem Medizingerät, aufweisend:
- eine Anzahl von Kabeln (10), und
- eine Anzahl von Klemmvorrichtungen (100) nach einem der vorherigen Ansprüche.

11. Sensorkabel (200) nach Anspruch 10,
wobei die Anzahl von Kabeln (10) und die Anzahl von Klemmvorrichtungen (100) integral miteinander ausgebildet sind.
